# EUROPEAN PATENT SPECIFICATION

(11) **EP 3 643 246 B1**
(45) Date of publication and mention of the grant of the patent: **28.12.2022**
(21) Application number: 18820722.9
(22) Date of filing: 25.06.2018
(51) Int. Cl.: A61B 8/08, G06N 99/00, G06K 9/62, G06N 3/02, G06N 7/00, G06N 20/00, G06N 3/04, G06N 3/08, G06T 7/00, G06T 7/11, G06V 10/44, G06V 10/82

(54) **METHOD FOR PROCESSING ULTRASONIC IMAGE**
VERFAHREN ZUR VERARBEITUNG EINES ULTRASCHALLBILDES
PROCÉDÉ DE TRAITEMENT D'IMAGE ULTRASONORE

(30) Priority: 23.06.2017 KR 20170079891; 22.06.2018 KR 20180072164
(43) Date of publication of application: 29.04.2020
(73) Proprietor: T3Q CO., LTD., Seoul 08389 (KR)
(72) Inventor: KIM, Eun, Na, Seoul 06616 (KR); KIM, Chong, Jai, Seoul 05392 (KR); SUNG, Chang, Ohk, Seongnam-si Gyeonggi-do 13559 (KR)
(74) Representative: Nederlandsch Octrooibureau
(86) International application number: PCT/KR2018/007140
(87) International publication number: WO 2018/236195

(56) References cited:
- JP-A- 2003 033 356
- JP-A- 2016 043 039
- KR-A- 20160 040 593
- KR-A- 20160 063 128
- KR-B1- 101 496 198
- LEI BAIYING ET AL: "Automatic placental maturity grading via hybrid learning", NEUROCOMPUTING, vol. 223, 27 October 2016 (2016-10-27), pages 86-102, XP029830494, ISSN: 0925-2312, DOI: 10.1016/J.NEUCOM.2016.10.033

## Description

### Technical Field

Example embodiments relate to a method of providing an ultrasonic image analysis, and more particularly, to a method of analyzing an ultrasound image, or a sonogram, of a pregnant woman in a gynecologic and obstetric diagnosis or examination and thereby facilitating diagnosis of a potential disease that the pregnant woman and her fetus may have.

### Background Art

An ultrasound or ultrasonography may be a pivotal means for medical use to diagnose a pregnant woman. This is because, for a pregnant woman and a fetus, administrating a medicine or drug is not available due to potential toxicity and fetal deformity, and applying a computed tomography (CT) is not available because a contrast agent is not allowed to be used. In addition, magnetic resonance imaging (MRI) is not available because a pregnant woman is highly likely to be exposed to a great deal of magnetic fields and loud noise while lying on her back with her inferior vena cava (IVC) being pressed. In some cases, MRI may use a gadolinium contrast agent, which may be restricted in use for its specificity.

Currently, a blood flow doppler method, in addition to a fetal ultrasound imaging that ensures safety and stability, is generally used to diagnose a potential disease that a pregnant woman and a fetus may have. Thus, there is a desire for a method of analyzing a correlation between an ultrasound image (or a sonogram) and an actual placental pathology, and diagnosing a disease that a fetus may have, only using the ultrasound image.

Document "Automatic placental maturity grading via hybrid learning" (NEUROCOMPUTING, vol. 223, pages 86-102), published on October 27, 2016, discloses a new method to automatically grade placental maturity from B-mode ultrasound (BUS) and color Doppler energy (CDE) images based on a hybrid learning architecture.

### Disclosure

### Technical Solutions

According to an example embodiment, there is provided an image processing method to be implemented by a computer, the image processing method including performing deep learning using a placental ultrasound image and a placental pathology image, separating a first area corresponding to a placenta from a received ultrasound image, extracting a first matching pathology image corresponding to the first area, and pathology image.

According to another example embodiment, there is provided an image processing method to be implemented by a computer, the image processing method including performing deep learning using a placental ultrasound image, a fetal ultrasound image, and a placental pathology image, separating a first area corresponding to a placenta from a received ultrasound image, separating a second area corresponding to a fetus from the received ultrasound image, extracting a first matching pathology image corresponding to the first area and the second area, and extracting at least one set of event information corresponding to the first matching pathology image.

The event information may include a disease information code mapped to the first matching pathology image.

The event information includes an estimated delivery date mapped to the first matching pathology image.

The image processing method may further include performing preprocessing to remove noise from the received ultrasound image.

The event information may include a disease information code mapped to the first matching pathology image.

According to still another example embodiment, there is provided an image processing apparatus configured to perform deep learning using a plurality of placental ultrasound images and placental pathology images, the image processing apparatus including a separator configured to separate a first area corresponding to a placenta from a received ultrasound image, and an extractor configured to extract a first matching pathology image corresponding to the first area, and at least one set of event information corresponding to the first matching pathology image.

According to yet another example embodiment, there is provided an image processing apparatus configured to perform deep learning using a placental ultrasound image, a fetal ultrasound image, and a placental pathology image, the image processing apparatus including a separator configured to separate, from a received ultrasound image, a first area corresponding to a placenta and a second area corresponding to a fetus, and an extractor configured to extract a first matching pathology image corresponding to the first area and the second area, and at least one set of event information corresponding to the first matching pathology image.

The event information may include a disease information code mapped to the first matching pathology image. The event information includes an estimated delivery date mapped to the first matching pathology image.

The image processing apparatus may further include a preprocessor configured to perform preprocessing to remove noise from the received ultrasound image.

According to further another example embodiment, there is provided an image processing apparatus configured to perform deep learning using a placental ultrasound image, a fetal ultrasound image, and a placental pathology image, the image processing apparatus including a separator configured to separate, from a received ultrasound image, a first area corresponding to a placenta and a second area corresponding to a fetus, and an extractor configured to extract first event information using at least one of pregnant woman data, biometric data, a placental ultrasound image, or a fetal ultrasound image, extract a first matching pathology image corresponding to the first area and the second area, and extract second event information corresponding to the first event information and the first matching pathology image.

According to further another example embodiment, there is provided an image processing method to be implemented by a computer, the image processing method including extracting first event information using at least one of pregnant woman data, biometric data, or an ultrasound image, performing deep learning using a placental ultrasound image, a fetal ultrasound image, and a placental pathology image, separating a first area corresponding to a placenta from a received ultrasound image, separating a second area corresponding to a fetus from the received ultrasound image, extracting a first matching pathology image corresponding to the first area and the second area, and extracting second event information corresponding to the first event information and the first matching pathology image.

### Brief Description of Drawings

FIG. 1 is a diagram illustrating a correlation between an ultrasound image and a placental pathology according to an example embodiment.
FIG. 2a is an ultrasound image obtained in a case of a cyst according to an example embodiment.
FIG. 2b is a placental microscopy image obtained in a case of a cyst according to an example embodiment.
FIG. 3a is an ultrasound image obtained in a case of hemorrhage according to an example embodiment.
FIG. 3b is a placental microscopy image obtained in a case of hemorrhage according to an example embodiment.
FIG. 4a is an ultrasound image obtained in a case of placental separation according to an example embodiment.
FIG. 4b is a placental microscopy image obtained in a case of placental separation according to an example embodiment.
FIG. 5 is a diagram illustrating a flow of an entire system according to an example embodiment.
FIG. 6 is a diagram illustrating a flow of a first assessment and a second assessment according to an example embodiment.
FIG. 7 is a diagram illustrating a flow of an example of identifying a presence or absence of a disease in a small fetus according to an example embodiment.
FIG. 8 is a diagram illustrating a flow of an example of an image processing method applied in an early stage of a pregnancy according to an example embodiment.
FIG. 9 is a diagram illustrating an example of a second assessment based on a first assessment according to an example embodiment.
FIG. 10 is a diagram illustrating an example of an algorithm for recommending an optimal delivery time according to an example embodiment.
FIG. 11 is a diagram illustrating an example of an algorithm for various situations according to an example embodiment.

### Best Mode for Carrying Out the Invention

Hereinafter, example embodiments will be described in detail with reference to the accompanying drawings. Regarding the reference numerals assigned to the elements in the drawings, it should be noted that the same elements will be designated by the same reference numerals, wherever possible, even though they are shown in different drawings.

The terminology used herein is for describing various examples only, and is not to be used to limit the disclosure. The features described herein may be embodied in different forms, and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application.

Unless otherwise defined, all terms, including technical and scientific terms, used herein have the same meaning as commonly understood by one of ordinary skill in the art to which this disclosure pertains based on an understanding of the present disclosure. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

### Necessity of Noninvasive Prenatal Diagnostic Method

When analyzing a placental pathology image, a morphologic method and an immunohistochemical method may be used to determine a presence or absence of a disease that a fetus may have, and a severity and a cause of the disease if any, by microscopically observing a placenta. However, the placenta may be obtained after delivery, and thus may not be readily used to diagnose the fetus during a pregnancy. Although a biopsy may be performed by isolating or sampling a portion of a placental tissue, the portion of the placental tissue may not represent the entire placenta, and thus may not be reliable.

In addition, echotexture of the placenta may be heterogenous, and thus may not be discriminable with a human eye. Further, there have been only a handful of studies and research on a relationship between a placental ultrasound image from placental ultrasonography and an actual placental pathology. This is because an obstetrician and/or gynecologist may have relatively less knowledge of placental pathologies, and a placental pathologist may have relatively less knowledge of ultrasound.

Thus, there is provided a noninvasive prenatal diagnostic method that analyzes a correlation between an ultrasound image (or sonogram) and an actual placental pathology only using the ultrasound image through artificial intelligence (AI)-based deep learning.

### Learning Pregnancy Result Using Ultrasound Image and Placental Pathology Image

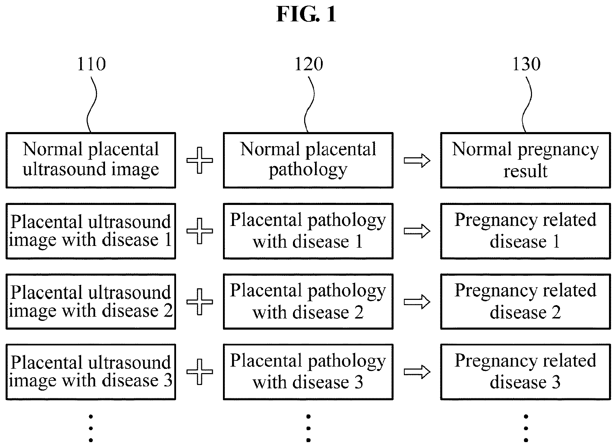

FIG. 1 is a diagram illustrating a pregnancy result corresponding to an ultrasound image and a placental pathology image according to an example embodiment. As illustrated, learning may be performed using a pregnancy result 130 corresponding to a placental ultrasound image 110 and a placental pathology image 120.

According to an example embodiment an image processing apparatus, which is also referred to herein as an ultrasound image processing apparatus, may perform the learning, for example, deep learning, using a plurality of placental ultrasound images and a plurality of placental pathology images. When performing the deep learning, pregnancy result information corresponding to an ultrasound image and a placental pathology image may be provided as input data. After learning a plurality of ultrasound images and placental pathology images through such deep learning, the image processing apparatus may extract a placental pathology image corresponding to an ultrasound image.

For example, the image processing apparatus may select a placental pathology image that matches the most a placental ultrasound image in which a cyst is found. In this example, the placental pathology image to be selected may have a highest correlation with the placental ultrasound image in which a cyst is found. The image processing apparatus may extract event information corresponding to the placental pathology image selected based on the placental ultrasound image obtained in a case of occurrence of a cyst. The event information may be information associated with a disease classification code of a disease identified from the placental pathology image, or information associated with a maintainable pregnancy period in which a pregnancy is maintained safely or stably, a desirable delivery time, and the like.

### Matching Placental Ultrasound Image and Placental Pathology Image

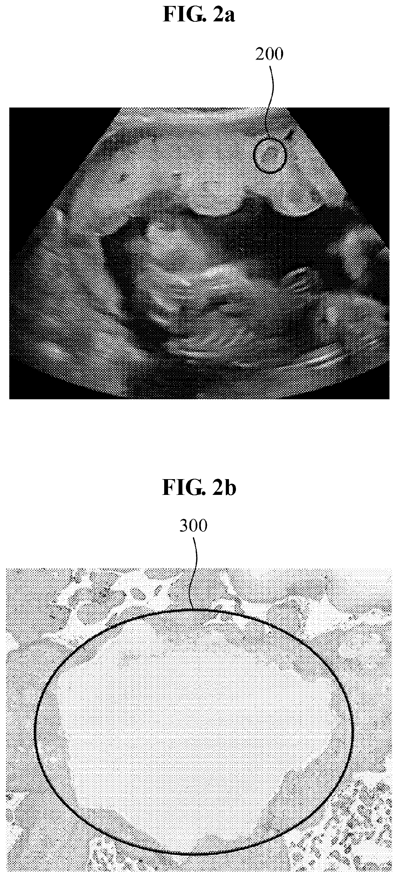

FIG. 2a is an ultrasound image obtained in a case of a cyst according to an example embodiment. A black portion of an indicated area 200 in an ultrasound image in FIG. 2a is where a cyst occurs. However, whether a cyst occurs or not may not be readily determined only using an ultrasound image. However, it may be determined that the cyst occurs in the block portion of the area 200 by analyzing a correlation with reference to FIG. 2b.

FIG. 2b is a placental microscopy image obtained in a case of a cyst according to an example embodiment. FIG. 2b is an example microscopy image obtained by observing a placenta after delivery or parturition. Dissimilar to a shape of a normal placenta, it may be determined that a cyst occurs. The cyst may be shown in a shape which is shown as in an internal portion of the area 200. Here, an analysis of a correlation between what is shown in FIG. 2a and what is shown in FIG. 2b may be learned through deep learning. A deep learning apparatus for which the learning is sufficiently performed may separate a placental area from the ultrasound image of FIG. 2a, and select a placental pathology image to be mapped to the placental area in response to the placental area being input. Here, the placenta area, for example, a first area corresponding to a placenta, may not necessarily be separated and used as an input. The entire ultrasound image may be input as needed, and a separator of an image processing apparatus may then separate an area corresponding to the placenta from the input ultrasound image.

According to another example embodiment, a first area corresponding to a placenta and a second area corresponding to a fetus may be respectively input. Here, a fetal ultrasound image may also be used. However, the first area and the second area may not be necessarily used as an input to the image processing apparatus. For example, when an entire ultrasound image is input, the separator of the image processing apparatus may separate an area corresponding to the placenta and an area corresponding to the fetus from the input ultrasound image, and select a pathology image to be mapped to the areas.

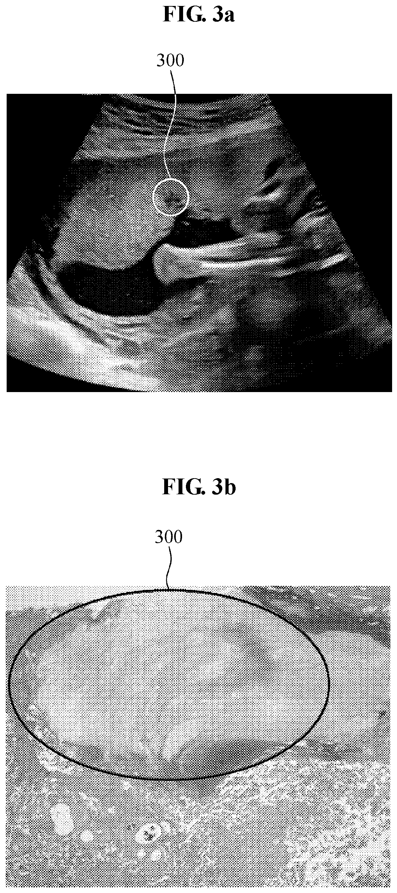

FIG. 3a is an ultrasound image obtained in a case of hemorrhage according to an example embodiment. There is a portion that is darker than a surrounding area in an indicated area 300 in FIG. 3a. A situation or state that may occur in such black portion may not be readily determined only using an ultrasound image. However, the black portion may be determined to be an area in which hemorrhage occurs with reference to an indicated area 300 in a placental pathology image of FIG. 3b. Thus, a correlation with what is shown in FIG. 3b may be analyzed.

FIG. 3b is a placental microscopy image obtained in a case of hemorrhage according to an example embodiment. It may be readily verified that hemorrhage occurs in a placenta based on an indicated area 300 in a placental microscopy image of FIG. 3b. Thus, an image processing apparatus may analyze a correlation between a circle portion indicated in a bold line in FIG. 3a and a circle portion indicated in a bold line in FIG. 3b.

When an ultrasound image similar to the ultrasound image of FIG. 3a or an area corresponding to a placenta that is extracted from the ultrasound image is input, the image processing apparatus that learns such information may select the image of FIG. 3b as a corresponding pathology image to be mapped.

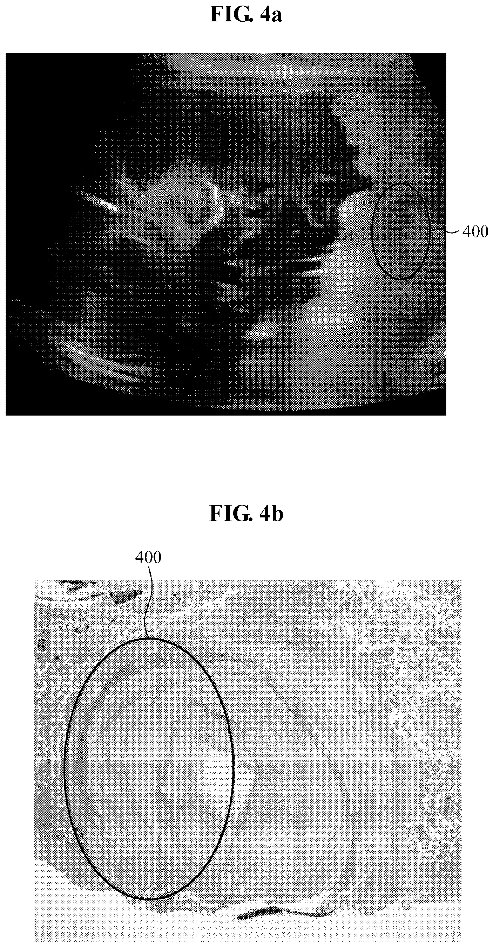

FIG. 4a is an ultrasound image obtained in a case of placental separation according to an example embodiment. FIG. 4b is a placental microscopy image obtained in a case of placental separation according to an example embodiment.

An image processing apparatus may analyze and learn a correlation between an ultrasound image associated with placental separation and a placental microscopy image associated with placental separation with reference to FIGS. 4a and 4b. An indicated area 400 in FIG. 4a corresponds to an indicated area 400 in FIG. 4b. When a deep learning apparatus (or the image processing apparatus as used herein) receives, as an input, an image similar to the ultrasound image associated with placental separation after the learning is completed, the deep learning apparatus may select the image of FIG. 4b as a corresponding pathology image to be mapped.

### AI-based Deep Learning and Extraction of Matching Pathology Image

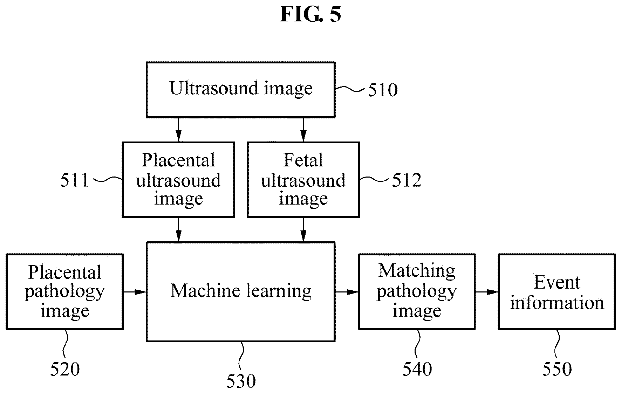

FIG. 5 is a diagram illustrating a flow of an operation of an image processing apparatus according to an example embodiment.

An image processing apparatus 530 may perform deep learning using an ultrasound image 510 and a placental pathology image 520. The ultrasound image 510 may be separated into a placental ultrasound image 511 and a fetal ultrasound image 512, and a correlation with the placental pathology image 520 may be analyzed. The image processing apparatus 530 may be trained to discover a pathology image corresponding to an ultrasound image.

An image processing method, which may also be referred to herein as an ultrasound image processing method, may be performed using an image processing apparatus trained through deep learning. A first area corresponding to the placental ultrasound image 511 may be extracted from a received ultrasound image, and be input to the image processing apparatus. The image processing apparatus trained through deep learning may extract a matching pathology image 540 corresponding to the first area, and event information corresponding to the matching pathology image 540. The event information may include a disease classification code or a desirable delivery time that corresponds to the matching pathology image 540, but not be limited thereto. The event information may include medical information corresponding to the matching pathology image 540.

When the image processing apparatus is learning the placental ultrasound image 511 and the placental pathology image 520, a convolution neural network (CNN) may be used to discover important features or characteristics to be used to diagnose a disease from an ultrasound image, through learning of comparative data indicating a comparison between an ultrasound image indicating the disease and a corresponding placental histopathological opinion.

For the features discovered as described above, a predictive model may be generated through artificial intelligence (AI)-based deep learning as post-processing. Here, an optimal combination of such various features may be discovered, and the predictive model may perform validation through, for example, 10-fold leave-one-out cross validation.

According to another example embodiment, when using a second area corresponding to the fetal ultrasound image 512 in addition to a first area corresponding to a placenta in an ultrasound image, the image processing apparatus may extract the matching pathology image 540 by combining variables associated with a fetus with the features obtained through the CNN In detail, the variables may be clinical variables measured from image information and include, for example, a height, a head circumference, a nuchal length, and a presence or absence of a nasal bone of the fetus, and the like. By combining such variables associated with the fetus and analyzing a correlation, it is possible to improve accuracy of the predictive model.

To use the fetal ultrasound image 512 in addition to the placental ultrasound image 511, various sets of additional information may be collected. Fundamentally, pregnant woman data, biometric data, and ultrasound fetal measurement data, may be collected. The pregnant woman data may include information associated with an age of a pregnant woman, a first date of a last menstruation period, a drug administration history, a past medical history, whether a pregnancy of the pregnant woman is a natural pregnancy, a pre-pregnancy hormonal state, a quad test, a visual abnormality, a headache, and the like. The biometric data may include information associated with a bimanual or combined examination result, a fetal heart rate, an uterine contraction monitoring result, a prenatal genetic test result, and the like. The ultrasound fetal measurement data may include information associated with a predicted weight, a leg length, a head circumference, an abdominal circumference, a biparietal diameter, and the like.

The image processing apparatus may generate an algorithm by matching an ultrasound image and a pathology image based on the pregnant woman data, the biometric data, and the ultrasound fetal measurement data, and on whether it is a high-risk pregnancy. In addition, the image processing apparatus may perform deep learning by categorizing potential diseases that may occur in a fetus.

To analyze a correlation between an ultrasound image and a placental pathology image, an optimal parameter may need to be discovered to generate a predictive model with a relatively high level of accuracy based on an extracted feature, and thus a cross-validation may be used. The parameter may be the number of hidden layers, for example. However, the parameter is not limited to the example, and any parameter that may be applicable to the predictive model may be used.

When the placental ultrasound image 511 is input to the image processing apparatus trained through deep learning, a first output may be related to whether toxemia of pregnancy occurs or not, or to placental separation, fetal infection, and the like, as needed. Since data is generated on a regular basis at an interval of several weeks, which is an advantage of prenatal ultrasound data, it is possible to recommend a desirable delivery date through a recurrent neural network (RNN).

### First Assessment and Second Assessment

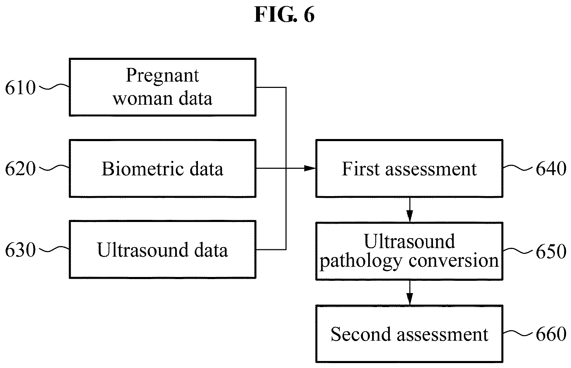

FIG. 6 is a diagram illustrating a flow of a first assessment and a second assessment according to an example embodiment. A first assessment 640 may be performed using sets of basic data 610, 620, and 630, and a second assessment 660 may be performed using ultrasound pathology conversion 650.

In detail, an image processing apparatus may perform the first assessment 640 using pregnant woman data 610, biometric data 620, and ultrasound data 630. In the first assessment 640, using the basic data, the image processing apparatus may predict or present a presence or absence of a potential disease, and assess stability of a pregnancy of a pregnant woman.

After the first assessment 640, the image processing apparatus may perform the ultrasound pathology conversion 650. In the ultrasound pathology conversion 650, the image processing apparatus may extract a matching pathology image using the ultrasound data 630. The image processing apparatus may then perform the second assessment 660 using the extracted matching pathology image. In the second assessment 660, the image processing apparatus may predict or present a potential fetal disease, a delivery time, and the like based on a result of the first assessment 640 and a result of the ultrasound pathology conversion 650.

### Identification of Small Fetus

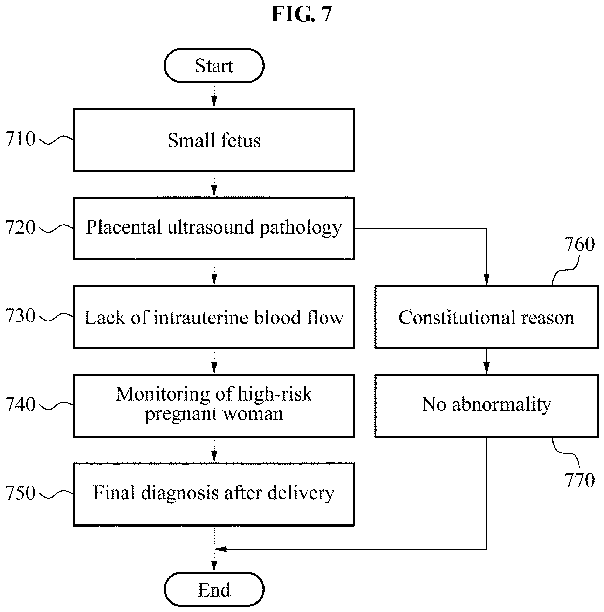

FIG. 7 is a diagram illustrating a flow of an example of identifying a presence or absence of a disease in a small fetus according to an example embodiment. For a small fetus 710, there may be a case in which a fetus does not normally grow due to a lack of intrauterine blood flow, and a case in which a fetus is simply physically or constitutionally small. Such two cases may be identified using a placental ultrasound pathology (algorithm) 720.

When a placental ultrasound image showing the small fetus 710 is input to an image processing apparatus, the image processing apparatus may extract a matching pathology image corresponding to the input placental ultrasound image. Using the extracted matching pathology image, whether the small fetus 710 is associated with a case 730 of a lack of intrauterine blood flow, or with a case 760 of a simple physical or constitutional reason may be identified. Based on the placental pathology image, the lack of intrauterine blood flow may be shown in a same or similar form as that of a placenta with toxemia of pregnancy, and this it may be identifiable.

When the small fetus 710 is associated with the simple physical or constitutional reason, such case 760 may be processed to be no abnormality found 770 and then terminated. However, when the case 730 of the lack of intrauterine blood flow is identified, a corresponding pregnant woman may be classified into a high-risk pregnant woman, and monitoring 740 of the high-risk pregnant woman may be performed. A final diagnosis 750 may then be made after delivery.

### Operation of Medical Service Using Image Processing Apparatus

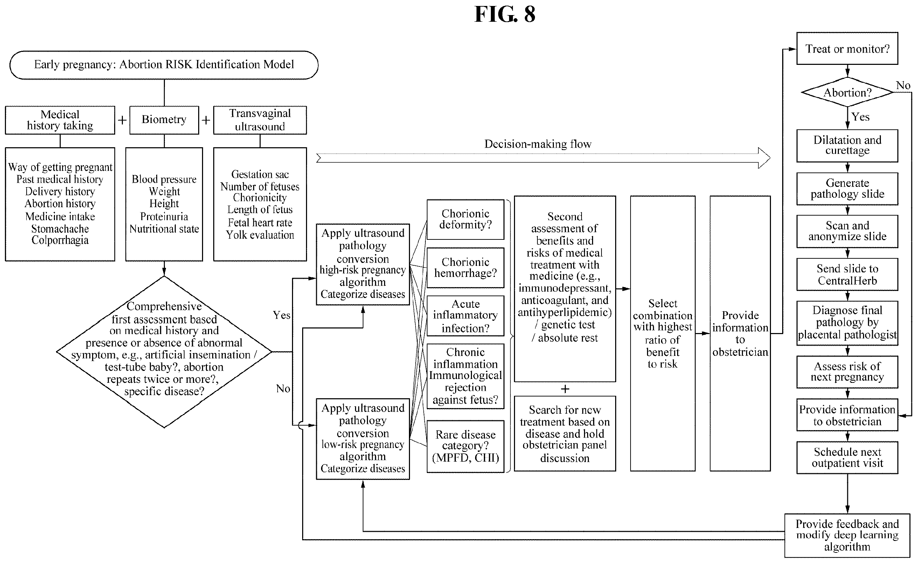

FIG. 8 is a diagram illustrating a flow of an example of an image processing method applied in an early stage of a pregnancy according to an example embodiment. An image processing method may be performed by verifying basic information associated with a pregnant woman using information associated with medial history taking, biometry, and transvaginal ultrasound, and the like, and by applying an ultrasound pathology conversion algorithm.

In detail, the information associated with the medical history taking may include information associated with, for example, a way of getting pregnant, a past medical history, a delivery history, an abortion history, a medicine intake, a stomachache, colporrhagia, and the like. The information associated with the biometry may include information associated with a blood pressure, a weight, a height, proteinuria, a nutritional state, and the like. The information associated with the transvaginal ultrasound may include information associated with a presence or absence of a gestation sac, the number of fetuses, a length of a fetus, a fetal heart rate, a yolk evaluation result, and the like. These sets of information may be comprehensively considered to perform a first assessment to determine whether there is an abnormal sign. Subsequently, which one between a high-risk pregnancy algorithm and a low-risk pregnancy algorithm may need to be applied may be determined.

Based on whether a pregnancy of the pregnant woman is a high-risk pregnancy or a low-risk pregnancy, the following-a presence or absence of chorionic deformity, chorionic hemorrhage, acute inflammatory infection, chronic inflammation, immunological rejection of the pregnant woman against a fetus, a rare disease, and the like-may be determined. Subsequently, a second assessment may be performed by considering benefits and risks of a medical treatment with a medicine such as, for example, immunodepressant, anticoagulant, and antihyperlipidemic, and of a genetic test and an absolute rest.

Based on the benefits and the risks, information associated with a combination having a highest benefit compared to a risk may be sent to a doctor. The doctor may then determine whether to treat or monitor the pregnant woman based on such received information.

When the pregnant woman aborts, dilatation and curettage may be performed, and a placenta obtained thereby may be used to generate a pathology slide. The generated slide may be scanned to obtain a corresponding pathology image, and anonymized to be sent to a central herb. The central herb may use such received placental pathology image to make a final diagnosis of a pathology, and assess a potential risk involved with a next pregnancy.

Information associated with such risk of a next pregnancy may be provided to an obstetrician. Here, when the pregnant woman does not abort or give birth, such pathology slide may not be generated, and the obstetrician may be informed that the pregnancy is to be maintained.

A schedule for a next outpatient visit may be arranged, and a deep learning algorithm may be modified or changed using a series of processes.

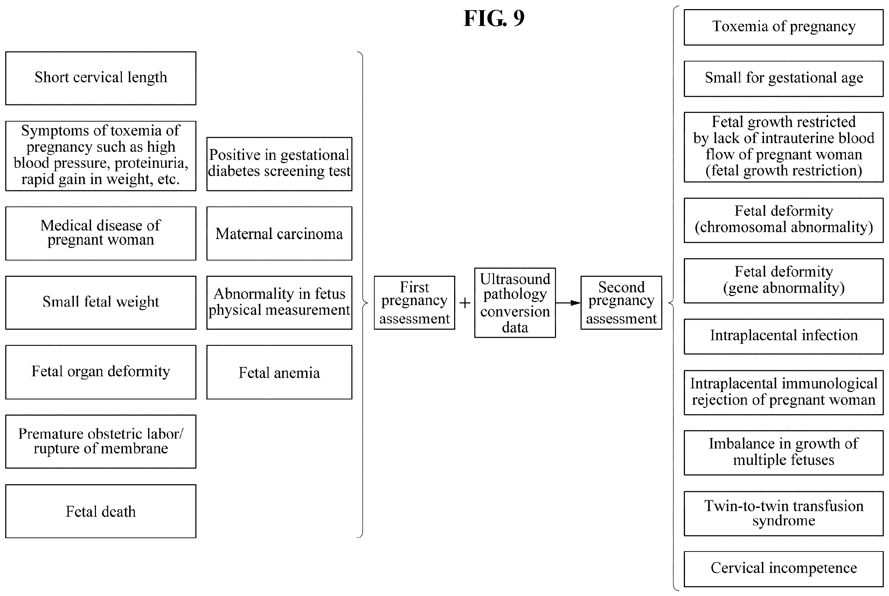

FIG. 9 is a diagram illustrating an example of a second assessment based on a first assessment according to an example embodiment. A first pregnancy assessment may be performed using information associated with a maternal carcinoma, a fetal organ deformity, a fetal anemia, and the like. A second pregnancy assessment may then be performed based on considerations that may be diagnosed by a placental change that is not applied to the first pregnancy assessment. The considerations in the second pregnancy assessment may include toxemia of pregnancy, an intraplacental infection, and intraplacental immunological rejection of a pregnant woman.

In detail, the first pregnancy assessment may be performed using information associated with a symptom or condition of a pregnant woman, a premature obstetric labor, a fetal body proportion, a cervical length, and the like, and may classify diseases to which an ultrasound pathology conversion-based high-risk pregnancy algorithm is applied. The diseases may be classified into five main categories and 22 subcategories. The main categories may include intrauterine infection and acute inflammation, decreased intrauterine blood flow, fetal vasoocclusion, immunological rejection of a pregnant woman against a fetus, and placental villus deformity.

The second pregnancy assessment may classify in more detail states of diseases that are not classified in the first pregnancy assessment and assess risks of the diseases, by applying a placental conversion algorithm.

What is to be assessed in the second pregnancy assessment may include, for example, placental separation, toxemia of pregnancy, a limited growth due to a lack of intrauterine blood flow, a fetal deformity due to chromosomal abnormality, a fetal deformity due to minor chromosomal abnormality or genetic mutation, an intraplacental infection, an intraplacental immunological rejection of a pregnant woman, imbalance in growth of multiple fetuses, twin-to-twin transfusion syndrome, cervical incompetence, deteriorating pregnancy-related diseases, and others.

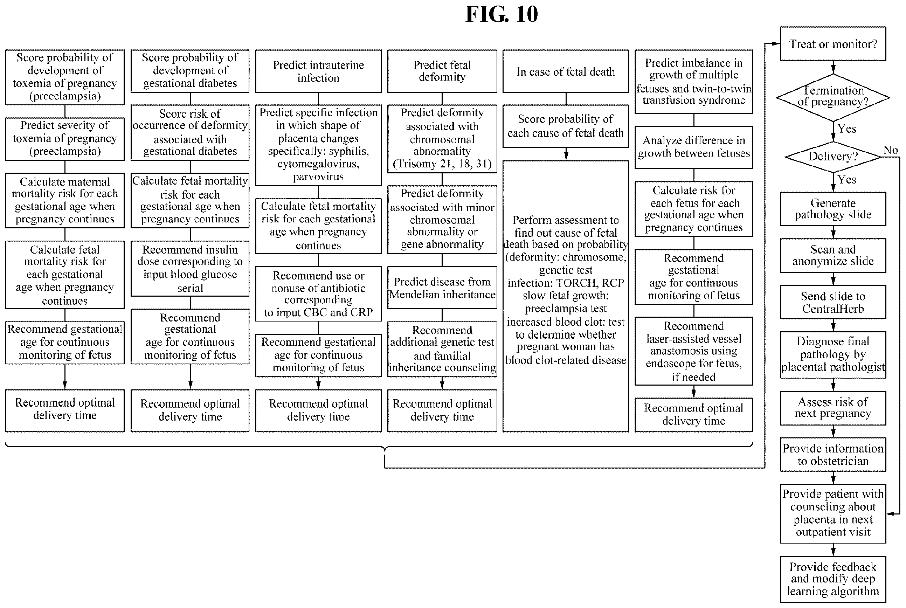

FIG. 10 is a diagram illustrating an example of an algorithm for recommending an optimal delivery time according to an example embodiment. By considering cases of toxemia of pregnancy, gestational diabetes, intrauterine infection, and the like, it is possible to recommend an optimal delivery time.

In detail, following operations may be performed for each of the cases. In a case of toxemia of pregnancy, operations to be performed may include scoring a probability of development of toxemia of pregnancy, predicting a severity of toxemia of pregnancy, calculating a maternal mortality rate and a fetal mortality risk for each gestational age when a pregnancy continues, and recommending a gestational age or pregnancy week from which on continuous fetal monitoring is needed, and finally recommending an optimal delivery time.

In a case of gestational diabetes, similar operations may also be performed. The operations may include scoring a risk of development of gestational diabetes, scoring a risk of occurrence of deformity associated with gestational diabetes, calculating a fetal mortality risk for each gestational age or pregnancy week, recommending an insulin dosage in response to a blood glucose level being continuously input, recommending a gestational age or pregnancy week from which on continuous fetal monitoring is needed, and recommending an optimal delivery time.

In a case of intrauterine infection, a specific infection in which a shape of a placenta changes specifically may be predicted. For example, the infection may include syphilis, cytomegalovirus (CMV) infection, parvovirus infection. In such case, similar operations may also be performed. The operations may include calculating a fetal mortality risk for each gestational age or pregnancy week when a pregnancy continues, recommending continuous use or nonuse of antibiotic in response to measurements or data such as a body temperature, a complete blood count (CBC), and a C-reactive protein (CRP) being input, recommending a gestational age or pregnancy week from which on continuous fetal monitoring is needed, and recommending an optimal delivery time.

For such various cases, an optimal delivery time may be recommended, and a corresponding treatment or tracking and observation (also referred to as monitoring herein) may be performed. As the optimal delivery time arrives, it is also possible to compare a placental ultrasound image obtained during a pregnancy and an actual placental microscopy image obtained by delivery, and provide feedback and modify a deep learning algorithm.

### Learning Algorithm for Various Situations

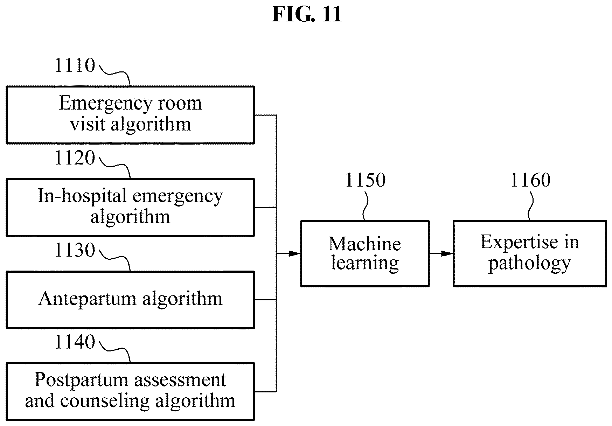

FIG. 11 is a diagram illustrating an example of an algorithm for various situations according to an example embodiment. For example, there may be algorisms for various situations that include, for example, an emergency room visit algorithm 1110, in-hospital emergency algorithm 1120, an antepartum algorithm 1130, and a postpartum assessment and counseling algorithm 1140. Through such algorithms to be applied to an image processing apparatus, learning 1150 may be performed, and expertise in pathology 1160 corresponding to a level of such expertise possessed by a pathologist may be provided to an obstetrician.

In such algorithms, nonstress test (NST) and Toco monitoring may also be learned or interpreted through deep learning, and be included in a risk assessment. The NST refers to a test used in a pregnancy to assess a relationship between a movement of a fetus and a heart rate under a condition without stress or stimulation. The Toco monitoring refers to a test to assess a relationship between uterine contraction and a fetal heart rate.

In addition, the image processing apparatus and method described herein may determine a recommended delivery date for twins. In a case of twins that are different in growth, a smaller fetus may need to be delivered promptly and a larger fetus may be delivered prematurely due to the smaller fetus. However, in a case of twin fetuses in a single chorion, if a pregnancy continues for a larger fetus, it may be highly likely that the larger fetus that survives from a death of a smaller fetus may suffer severe brain damage. Thus, the pregnancy many need to be maintained to the maximum period until the smaller fetus may survive without being dead.

To determine the recommended delivery date for twins, a placental ultrasound image may also be used. To this end, a placental growth may be scored, and a deep learning apparatus may determine an optimal delivery time.

Although some example diseases have been described above in relation to a placental image, related diseases may not be limited to the example diseases and may include, for example, a placental metastasis of a cancerous tumor of a pregnant woman and a fetus, a congenital rare metabolic disorder, a fetal infection, an intrauterine fetal death, a placental deformity, and the like.

The units described herein may be implemented using hardware components and software components. For example, the hardware components may include microphones, amplifiers, band-pass filters, audio to digital converters, non-transitory computer memory and processing devices. A processing device may be implemented using one or more general-purpose or special purpose computers, such as, for example, a processor, a controller and an arithmetic logic unit (ALU), a digital signal processor, a microcomputer, a field programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device.

The methods according to the above-described example embodiments may be recorded in non-transitory computer-readable media including program instructions to implement various operations of the above-described example embodiments. The media may also include, alone or in combination with the program instructions, data files, data structures, and the like. The program instructions recorded on the media may be those specially designed and constructed for the purposes of example embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher level code that may be executed by the computer using an interpreter. The above-described devices may be configured to act as one or more software modules in order to perform the operations of the above-described example embodiments, or vice versa.

While this disclosure includes specific examples, it will be apparent to one of ordinary skill in the art that various changes in form and details may be made in these examples without departing from the scope of the claims.

The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents.

Therefore, the scope of the disclosure is defined not by the detailed description, but by the claims.

## Claims

1. An image processing method to be implemented by a computer, comprising:
performing deep learning using a placental ultrasound image (511) and a placental pathology image (520);
separating a first area corresponding to a placenta from a received ultrasound image (510);
extracting a first matching pathology image (540) corresponding to the first area; and
extracting at least one set of event information corresponding to the first matching pathology image (540),
wherein the event information is an estimated delivery date.

2. The image processing method of claim 1, further comprising:
performing preprocessing to remove noise from the received ultrasound image (510) .

3. The image processing method of claim 1, wherein the performing deep learning comprises performing deep learning using a placental ultrasound image (511), a fetal ultrasound image (512), and a placental pathology image (520).

4. The image processing method of claim 1, wherein the extracting the first matching pathology image (540) comprises:
separating a second area corresponding to a fetus from the received ultrasound image (510); and
extracting the first matching pathology image (540) corresponding to the first area and the second area.

5. The image processing method of claim 1, further comprising:
extracting first event information using at least one of pregnant woman data, biometric data, or an ultrasound image

6. The image processing method of claim 5, wherein extracting at least one set of event information comprises extracting second event information corresponding to the first event information and the first matching pathology image (540).

7. An image processing apparatus configured to perform deep learning using a plurality of placental ultrasound images (511) and placental pathology images (520), the image processing apparatus comprising:
a separator configured to separate a first area corresponding to a placenta from a received ultrasound image (510); and
an extractor configured to extract a first matching pathology image (540) corresponding to the first area, and at least one set of event information corresponding to the first matching pathology image (540),
wherein the event information is an estimated delivery date.

8. The image processing apparatus of claim 7, further comprising:
a preprocessor configured to perform preprocessing to remove noise from the received ultrasound image (510).

9. The image processing apparatus of claim 7, wherein the separator is further configured to separate, from the received ultrasound image (510), the first area corresponding to the placenta and a second area corresponding to a fetus; and
the extractor is further configured to extract the first matching pathology image (540) corresponding to the first area and the second area, and at least one set of event information corresponding to the first matching pathology image (540).

10. The image processing apparatus of claim 9, wherein the extractor is further configured to extract first event information using at least one of pregnant woman data, biometric data, a placental ultrasound image (511), or a fetal ultrasound image (512).

11. The image processing apparatus of claim 10, wherein the extractor is further configured to extract second event information corresponding to the first event information and the first matching pathology image (540).

## Patentansprüche

1. Bildverarbeitungsverfahren zur Implementierung durch einen Computer, umfassend:
Durchführen von Deep Learning unter Verwendung eines plazentaren Ultraschallbildes (511) und eines plazentaren Pathologiebildes (520);
Trennen eines ersten Bereichs, der einer Plazenta entspricht, aus einem empfangenen Ultraschallbild (510);
Extrahieren eines ersten übereinstimmenden Pathologiebildes (540), das dem ersten Bereich entspricht; und
Extrahieren mindestens eines Satzes von Ereignisinformationen entsprechend dem ersten übereinstimmenden Pathologiebild (540),
wobei die Ereignisinformation ein geschätztes Entbindungsdatum ist.

2. Bildverarbeitungsverfahren nach Anspruch 1, das ferner umfasst:
Durchführen einer Vorverarbeitung zum Entfernen von Rauschen aus dem empfangenen Ultraschallbild (510).

3. Bildverarbeitungsverfahren nach Anspruch 1, wobei das Durchführen von Deep Learning das Durchführen von Deep Learning unter Verwendung eines plazentaren Ultraschallbildes (511), eines fötalen Ultraschallbildes (512) und eines plazentaren Pathologiebildes (520) umfasst.

4. Bildverarbeitungsverfahren nach Anspruch 1, wobei das Extrahieren des ersten übereinstimmenden Pathologiebildes (540) umfasst:
Abtrennen eines zweiten Bereichs, der einem Fötus entspricht, aus dem empfangenen Ultraschallbild (510); und
Extrahieren des ersten übereinstimmenden Pathologiebildes (540), das dem ersten Bereich und dem zweiten Bereich entspricht.

5. Bildverarbeitungsverfahren nach Anspruch 1, das ferner umfasst:
Extrahieren erster Ereignisinformationen unter Verwendung von mindestens einem von Daten einer schwangeren Frau, biometrische Daten oder ein Ultraschallbild.

6. Bildverarbeitungsverfahren nach Anspruch 5, wobei das Extrahieren mindestens eines Satzes von Ereignisinformationen das Extrahieren zweiter Ereignisinformationen umfasst, die den ersten Ereignisinformationen und dem ersten übereinstimmenden Pathologiebild (540) entsprechen.

7. Bildverarbeitungsvorrichtung, die so konfiguriert ist, dass sie Deep Learning unter Verwendung von mehreren plazentaren Ultraschallbildern (511) und plazentaren Pathologiebildern (520) durchführt, wobei die Bildverarbeitungsvorrichtung umfasst:
einen Separator, der so konfiguriert ist, dass er einen ersten Bereich, der einer Plazenta entspricht, aus einem empfangenen Ultraschallbild (510) trennt; und
einen Extraktor, der so konfiguriert ist, dass er ein erstes übereinstimmendes Pathologiebild (540), das dem ersten Bereich entspricht, und mindestens einen Satz von Ereignisinformationen, die dem ersten übereinstimmenden Pathologiebild (540) entsprechen, extrahiert,
wobei die Ereignisinformation ein geschätztes Entbindungsdatum ist.

8. Bildverarbeitungsvorrichtung nach Anspruch 7, die ferner umfasst:
einen Vorprozessor, der so konfiguriert ist, dass er eine Vorverarbeitung durchführt, um Rauschen aus dem empfangenen Ultraschallbild (510) zu entfernen.

9. Bildverarbeitungsvorrichtung nach Anspruch 7, wobei der Separator ferner so konfiguriert ist, dass er aus dem empfangenen Ultraschallbild (510) den ersten Bereich, der der Plazenta entspricht, und einen zweiten Bereich, der einem Fötus entspricht, trennt; und
der Extraktor ferner so konfiguriert ist, dass er das erste übereinstimmende Pathologiebild (540), das dem ersten Bereich und dem zweiten Bereich entspricht, und mindestens einen Satz von Ereignisinformationen, der dem ersten übereinstimmenden Pathologiebild (540) entspricht, extrahiert.

10. Bildverarbeitungsvorrichtung nach Anspruch 9, wobei der Extraktor ferner so konfiguriert ist, dass er erste Ereignisinformationen unter Verwendung von mindestens einem von Daten einer schwangeren Frau, biometrischen Daten, einem plazentaren Ultraschallbild (511) oder einem fötalen Ultraschallbild (512) extrahiert.

11. Bildverarbeitungsvorrichtung nach Anspruch 10, wobei der Extraktor ferner so konfiguriert ist, dass er zweite Ereignisinformationen extrahiert, die den ersten Ereignisinformationen und dem ersten übereinstimmenden Pathologiebild (540) entsprechen.

## Revendications

1. Procédé de traitement d'image devant être mis en œuvre par un ordinateur, comprenant les étapes consistant à :
effectuer un apprentissage profond en utilisant une image ultrasonore placentaire (511) et une image de pathologie placentaire (520) ;
séparer une première zone correspondant à un placenta à partir d'une image ultrasonore reçue (510) ;
extraire une première image de pathologie de correspondance (540) correspondant à la première zone ; et
extraire au moins un ensemble d'informations d'événement correspondant à la première image de pathologie de correspondance (540),
dans lequel les informations d'événement sont une date d'accouchement estimée.

2. Procédé de traitement d'image selon la revendication 1, comprenant en outre l'étape consistant à :
effectuer un prétraitement pour éliminer du bruit à partir de l'image ultrasonore reçue (510).

3. Procédé de traitement d'image selon la revendication 1, dans lequel la réalisation d'un apprentissage profond consiste à effectuer un apprentissage profond en utilisant une image ultrasonore placentaire (511), une image ultrasonore fœtale (512), et une image de pathologie placentaire (520).

4. Procédé de traitement d'image selon la revendication 1, dans lequel l'extraction de la première image de pathologie de correspondance (540) comprend les étapes consistant à :
séparer une seconde zone correspondant à un fœtus à partir de l'image ultrasonore reçue (510) ; et
extraire de la première image de pathologie correspondante (540) correspondant à la première zone et à la seconde zone.

5. Procédé de traitement d'image selon la revendication 1, comprenant en outre l'étape consistant à :
extraire des premières informations d'événement en utilisant au moins certaines parmi des données de femme enceinte, des données biométriques ou une image ultrasonore.

6. Procédé de traitement d'image selon la revendication 5, dans lequel l'extraction d'au moins un ensemble d'informations d'événement comprend une extraction de secondes informations d'événement correspondant aux premières informations d'événement et à la première image de pathologie de correspondance (540).

7. Appareil de traitement d'image configuré pour effectuer un apprentissage profond en utilisant une pluralité d'images ultrasonores placentaires (511) et d'images de pathologie placentaire (520), l'appareil de traitement d'image comprenant :
un séparateur configuré pour séparer une première zone correspondant à un placenta d'une image ultrasonore reçue (510) ; et
un extracteur configuré pour extraire une première image de pathologie correspondante (540) correspondant à la première zone, et au moins un ensemble d'informations d'événement correspondant à la première image de pathologie de correspondance (540),
dans lequel les informations d'événement sont une date d'accouchement estimée.

8. Appareil de traitement d'image selon la revendication 7, comprenant en outre :
un préprocesseur configuré pour effectuer un prétraitement afin d'éliminer du bruit à partir de l'image ultrasonore reçue (510).

9. Appareil de traitement d'image selon la revendication 7, dans lequel le séparateur est en outre configuré pour séparer, à partir de l'image ultrasonore reçue (510), la première zone correspondant au placenta et une seconde zone correspondant à un foetus ; et
l'extracteur est en outre configuré pour extraire la première image de pathologie de correspondance (540) correspondant à la première zone et à la seconde zone, et au moins un ensemble d'informations d'événement correspondant à la première image de pathologie de correspondance (540).

10. Appareil de traitement d'image selon la revendication 9, dans lequel l'extracteur est en outre configuré pour extraire des premières informations d'événement en utilisant au moins certaines parmi des données de femme enceinte, des données biométriques, une image ultrasonore placentaire (511) ou une image ultrasonore fœtale (512).

11. Appareil de traitement d'image selon la revendication 10, dans lequel l'extracteur est en outre configuré pour extraire des secondes informations d'événement correspondant aux premières informations d'événement et à la première image de pathologie de correspondance (540).
